(19) Europäisches Patentamt
European Patent Office
Office européen des brevets

(11) Veröffentlichungsnummer: **0 203 462**
**B1**

(12) **EUROPÄISCHE PATENTSCHRIFT**

(45) Veröffentlichungstag der Patentschrift: **01.08.90**

(21) Anmeldenummer: **86106573.8**

(22) Anmeldetag: **14.05.86**

(51) Int. Cl.$^5$: **C 07 C 69/653**, C 07 C 67/14, C 07 C 67/31, C 07 C 51/377, C 07 C 51/60, C 07 C 57/54, C 08 F 20/24

# (54) alpha-Fluoracrylsäureester und deren Polymere.

(30) Priorität: **25.05.85 DE 3518893**
**25.01.86 DE 3602275**
**30.04.86 DE 3614695**

(43) Veröffentlichungstag der Anmeldung:
**03.12.86 Patentblatt 86/49**

(45) Bekanntmachung des Hinweises auf die Patenterteilung:
**01.08.90 Patentblatt 90/31**

(84) Benannte Vertragsstaaten:
**AT BE CH DE FR GB IT LI LU NL SE**

(56) Entgegenhaltungen:
**DE-A-2 062 817**
**US-A-3 823 171**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Heumüller, Rudolf, Dr.**
**Sodener Weg 1**
**D-6232 Bad Soden am Taunus (DE)**
Erfinder: **Siegemund, Günter, Dr.**
**Frankfurter Strasse 21**
**D-6238 Hofheim am Taunus (DE)**
Erfinder: **Groh, Werner, Dr.**
**Geisenheimer Strasse 93**
**D-6000 Frankfurt am Main 71 (DE)**
Erfinder: **Wieners, Gerhard, Dr.**
**Unterweg 17**
**D-6000 Frankfurt am Main (DE)**
Erfinder: **Herbrechtsmeier, Peter, Dr.**
**Friedrich-Stolze-Strasse 10**
**D-6240 Königstein/Taunus (DE)**

**EP 0 203 462 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) Entgegenhaltungen:
G. Modena et al.: "The chemistry of the carbon-halogen bond", S. 306, S. Patai, John Wiley & Sons, London, 1973

B.E. Smart, "The chemistry of halides, pseudohalides and azides" (Supplement D), S. 614, S. Patai, John Wiley & Sons, London 1983
W.H. Dawson et al., J. Chem.Soc.Chem.Comm. 1980, S.875

**Beschreibung**

Die Erfindung bezieht sich auf α-Fluoracrylsäureester und deren Polymere, Verfahren zur Herstellung von α-Fluoracrylsäureestern und deren Polymeren sowie die Verwendung dieser Polymeren.

Ester der α-Fluoracrylsäure sind bereits bekannt. So wird der Phenylester der α-Fluoracrylsäure dadurch hergestellt, daß Monofluoressigsäureethylester in Gegenwart von Natriumethylat mit Ethoxyoxalat umgesetzt wird, das erhaltene Natrium-α-fluoracrylat mit Thionylchlorid in α-Fluoracryloylchlorid umgewandet wird und letzteres dann mit Phenol verestert wird (deutsche Patentschrift 29 50 491 = US-Patentschrift 4 297 466). Hierbei ist nachteilig, daß der hochtoxische Monofluoressigsäreethylester eingesetzt werden muß. Der α-Fluoracrylsäurephenylester ist polymerisierbar und dient zur Herstellung von Polymeren, die bei Raumtemperatur transparente bzw. durchsichtige oder lichtdurchlässige und farblose Feststoffe darstellen.

Weitere Ester der α-Fluoracrylsäure, insbesondere Butyl-α-fluoracrylat, sind herstellbar durch saure Hydrolyse des jeweiligen α-Hydroxymethyl-α-fluormalonats und anschließende Decarboxylierung des Hydrolyseproduktes unter gleichzeitiger Alkoholabspaltung (britische Patentschrift 1 115 287). Allerdings ist diese Methode nur am Beispiel des Butyl-α-fluoracrylats beschrieben; unter Lichteinfluß polymerisiert der Ester rasch.

Ferner ist bekannt, daß Polymere von α-Halogenacrylsäureestern mit halogenhaltigen Alkoholkomponenten zur Herstellung von strahlungsempfindlichen Schutzschichten dienen (US-Patentschrift 4 259 407). Als Ausgangsmaterial werden Monomere der Formel $H_2C = CX—COOR$ verwendet, in der X ein Fluor-, Chlor- oder Bromatom ist und R eine fluorierte Alkyl-, Aryl- oder Alkoxygruppe darstellt. Unter den Polymeren, die als Halogen nur Fluor enthalten, ist beispielsweise Poly(trifluorisopropyl-α-fluoracrylat erwähnt; über irgendwelche Eigenschaften dieses Polymers oder des entsprechenden Monomers sind jedoch keine Angaben vorhanden.

Schließlich ist ein optisches Material bekannt, das aus einem polymeren α-Fluoracrylsäureester besteht, der sowohl am β-Kohlenstoffatom der Vinylgruppe als auch in der Alkoholkompente Deuteriumatome enthalten kann (Europäische Anmeldungsveröffentlichung 0 128 517). Diese Polymeren dienen als Kernmaterial für optische Fasern; sie zeigen ein Molekulargewicht von 200 000 bis 5 000 000 (Gelpermeation), einen Brechungsindex von 1,45 bis 1,60 und eine Erweichungstemperatur von 100 bis 200°C. Als Mantelmaterial der optischen Fasern werden Polymere verwendet, die einen niedrigeren Brechungsindex aufweisen; hiefür eignen sich beispielsweise Polymere von α-Fluoracrylsäureestern, deren Alkoholkomponente Fluoratome enthält, z.B. Trifluorethyl-α-fluoracrylat und Hexafluorisobutyl-α-fluoracrylat.

Herstellung und Eigenschaften der vorgenannten Poly(fluoralkyl-α-fluoracrylate) sind ebenfalls beschrieben (Europäische Anmeldungsveröffentlichung 0 128 516). Die Polymere werden erhalten durch radikalisch initiierte Polymerisation der Monomeren in Masse, Lösung oder Suspension in Gegenwart eines Kettenübertragungsmittels bei einer Temperatur von 0 bis 100°C. Die Polymeren zeigen ein Molekulargewicht von 200 000 bis 5 000 000 (Gelpermeation), einen Brechungsindex von 1,36 bis 1,44 und eine Erweichungstemperatur von 80 bis 140°C.

Aufgabe der Erfindung ist die Bereitstellung von Estern der α-Fluoracrylsäure mit hochfluoriertem Alkoholrest sowie der entsprechenden Polymeren, die sich zu Gegenständen mit hoher Transparenz verarbeiten lassen.

Die Erfindung betrifft α-Fluoracrylsäureester der Formel (1)

(1) $$CH_2{=}CF{—}CO{—}O{—}C(CF_3)_2{—}R,$$

in der R ein Wasserstoffatom, ein Deuteriumatom, ein Halogenatom, einen aliphatischen Rest mit 1 bis 4 Kohlenstoffatomen oder einem aromatischen Rest mit 4 bis 10 Kohlenstoffatomen bedeutet; hierzu gehört beispielsweise der α-Fluoracrylsäure-hexafluorisopropylester der Formel (2)

(2) $$CH_2{=}CF{—}CO{—}O{—}C(CF_3)_2{—}H.$$

Die Erfindung betrifft ferner ein Verfahren zur Herstellung eines α-Fluoracrylsäureesters, das dadurch gekennzeichnet ist, daß in einem ersten Verfahrensschritt α-Fluormalonsäure-dimethylester mit Formaldwehyd umgesetzt wird, dann in einem zweiten Verfahrensschritt der erhaltene hydroxymethylierte α-Fluormalonsäure-dimethylester hydrolysiert, decarboxyliert und dehydratisiert wird und anschließend in einem dritten Verfahrensschritt die erhaltene α-Fluoracrylsäure, gegebenenfalls in Form eines Säurehalogenids, mit einem Alkohol der Formel (3)

(3) $$HO{—}C(CF_3)_2{—}R,$$

in der die bei Formel (1) angegebene Bedeutung hat, gegebenenfalls in Form eines Alkalialkoholats, verestert wird.

Weiterhin betrifft die Erfindung ein fluorhaltiges Polymer, das dadurch gekennzeichnet ist, daß es im wesentlichen aus Monomereinheiten besteht, die sich von einem α-Fluoracrylsäureester der Formel (1) ableiten.

Die Erfindung betrifft außerdem ein Verfahren zur Herstellung eines fluorhaltigen Polymers durch radikalisch initiierte Polymerisation eines fluorhaltigen Monomers, das dadurch gekennzeichnet ist, daß ein α-Fluoracrylsäureester der Formel (1), gegebenenfalls im Gemisch mit einer anderen, copolymerisierbaren Vinylverbindung, bei einer Temperatur von 60 bis 150°C polymersiert wird.

Schließlich betrifft die Erfindung noch die Verwendung eines fluorhaltigen Polymers, das im wesentlichen aus Monomereinheiten besteht, die sich von einem α-Fluoracrylsäureester der Formel (1) ableiten, als Material zur Herstellung von transparenten Gegenständen.

Das erfindungsgemäße Verfahren zur Herstellung eines α-Fluoracrylsäureesters wird in drei Stufen durchgeführt: Zunächst wird α-Fluoracrylsäure-dimethylester mit Formaldehyd zu α-Hydroxymethyl-α-fluormalonsäuredimethylester umgesetzt, dieser wird dann hydrolysiert und das Hydrolyseprodukt wird decarboxyliert und dehydratisiert, und schließlich wird die erhaltene α-Fluoracrylsäure mit einem Alkohol der Formel (3) verestert.

Im ersten Verfahrensschritt wird α-Fluormalonsäure-dimethylester einer Hydroxymethylierung mit Formaldehyd unterworfen. (α-Fluormalonsäure-dimethylester ist eine bekannte Verbindung; s. Journal of Fluorine Chemistry *25* (1984), 203—212.) Der Formaldehyd wird vorzugsweise in Form einer wäßrigen Lösung eingesetzt, die einen Formaldehydgehalt von 30 bis 40 Gewichtsprozent aufweist. Der Formaldehyd wird in einer Menge von 1 bis 10 mol, vorzugsweise 1,1 bis 3 mol, eingesetzt, (bezogen auf 1 mol α-Fluormalonsäure-dimethylester). Statt Formaldehyd kann auch Paraformaldehyd, Hexamethylentetramin oder 1,3,5-Trioxan verwendet werden. Es ist vorteilhaft, die Umsetzung im Gegenwart eines basischen Katalysators durchzuführen, der dann in einer Menge von 2 bis 50, vorzugsweise von 5 bis 15 Molprozent (bezogen auf den α-Fluormalonsäure-dimethylester) verwendet wird. Als Katalysator dient insbesondere eine Alkalihydrogencarbonat, z.B. Kaliumhydrogencarbonat und Natriumhydrogencarbonat. Die Reaktion wird bei einer Temperatur von 5 bis 40°C, vorzugsweise von 15 bis 30°C durchgeführt. Der enstandene α-Hydroxymethyl-α-fluormalonsäure-dimethylester wird anschließend aus dem Reaktionsgemisch isoliert, vorzugsweise durch Aussalzen oder Extraktion mit Hilfe eines mit Wasser nicht mischbaren organischen Lösungsmittels. Als Lösungsmittel eignet sich vor allem ein aliphatischer Chlorkohlenwasserstoff mit 1 bis 4 Kohlenstoffatomen, z.B. Dichlormethan, trichlormethan, Tetrachlormethan, 1,1-Dichlorethan, 1,2-Dichlorethan. Besonders vorteilhaft ist eine Kombination von Aussalzen und Extraktion; dabei wird das Reaktionsgemisch zunächst mit einer gesättigen Salzlösung (Ammomiumsulfat, Natriumchlorid) versetzt und dieses Gemisch wird dann extrahiert. Durch Verdampfen des Lösungsmittels wird α-Hydroxymethyl-α-fluormalonsäure als farbloser Festoff gewonnen.

In zweiten Verfahrensschritt wird der α-Hydroxymethyl-α-fluormalonsäure-dimethylester in einem wäßrigen sauren Medium hydrolysiert, und das Hydrolyseprodukt wird decarboxyliert und dehydratisiert. Die Reaktion wird bei einem pH-Wert von -1 bis 6, vorzugsweise 0 bis 2, durchgeführt; das saure Milieu wird mit Hilfe einer wäßrigen Säurelösung hergestellt, vorzugsweise einer verdünnten anorganischen Säure wie Salzsäure oder Schwefelsäure. Die Reaktionstemperatur liegt im Bereich von 90 bis 110°C, vorzugsweise 95 bis 105°C. Nach Beendigung der Gasentwicklung wird das Reaktionsgemisch bei einem Druck von 1013 bis 600 mbar destilliert, und das Destillat wird mit einem organischen Lösungsmittel extrahiert. Als Lösungsmittel wird hier ebenfalls ein mit Wasser nicht mischbares Lösungsmittel verwendet, vorzugsweise ein Ether wie Diethylether. Nach Verdampfen des Lösungsmittels wird α-Fluoracrylsäure als farbloser Feststoff erhalten. In einer bevorzugten Variante wird die α-Fluoracrylsäure als Ammoniumsalz isoliert. Dazu wird durch die nach der Extraktion erhaltene Lösung gasförmiges Ammoniak geleitet, und der farblose kristalline Niederschlag wird dann vom Lösungsmittel befreit.

Im dritten Verfahrensschritt die α-Fluoracrylsäure mit einem Alkohol der Formel (3) verestert. Der Alkohol wird in einer Menge von 0,5 bis 2 mol, vorzugsweise 0,8 bis 1,2 mol, eingesetzt (bezogen auf 1 mol α-Fluoracrylsäure). Für die Veresterung wird die α-Fluoracrylsäure als solche oder vorzugsweise in Form eines Säurehalogenids, insbesondere als α-Fluoracrylsäurechlorid, eingesetzt. Das Säurehalogenid wird mit Hilfe eines üblichen Halogenierungsmittels hegestellt z.B. Oxalylchlorid, Phosphorpentachlorid, Phosphortrichlorid, Phosphoroxychlorid, Benzoylchlorid, Benzotrichlorid, Phosphortribromid, Schwefeltetrafluorid und insbesondere Thionylchlorid. Die Halogenierung mit Thionylchlorid erfolgt vorzugsweise in Gegenwart eines Katalysators wie Dimethylformamid. Die Umsetzung wird in eine, aromatischen Kohlenwasserstoff, z.B. Toluol, Xylol und Trimethylbenzol, als Lösungsmittel durchgeführt, und die Reaktionstemperatur liegt im Bereich von 50 bis 100°C vorzugsweise 70 bis 90°C. Die Veresterung wird ebenfalls in einem Lösungsmittel durchgeführt, und die Reaktionstemperatur beträgt hier 0 bis 30°C, vorzugsweise 5 bis 25°C. Als Lösungsmittel eignen sich aliphatische, alicyclische oder aromatische Kohlenwasserstoffe, z.B. n-Hexan, n-Heptan, Cyclohexan, Methylcyclohexan, Benzol, Toluol, Xylol und Mesitylen, ferner symmetrische, asymmetrische oder cyclische Ether, z.B. Diethylether, Dipropylether, Diisopropylether, tert.Butylmethylether, Diethylenglykoldimethylether, Tetrahydrofuran und Dioxan, sowie aliphatische oder aromatische Halogenkohlenwasserstoffe, vorzugsweise Chlorkohlenwasserstoffe, z.B. Dichlormethan, Trichlormethan, Tetrachlormethan, 1,1-Dichlorethan, 1,2-Dichlorethan, Chlorbenzol und 1,2-Dichlorbenzol, und auch aliphatische oder aromatische Nitrile, z.B. Acetonitril und Benzonitril. Es ist zweckmäßig, die Veresterung des Säurehalogenids in Gegenwart einer organischen Base durchzuführen, insbesondere eines Trialkylamins mit jeweils 1 bis 4 Kohlenstoffatom in den Alkylgruppen, z.B. Triethylamin, Triisopropylamin und Tributylamin. Die Base wird in einer Menge von 0,5 bis 2 mol, vorzugsweise 0,8 bis 1,2 mol, eingesetzt (bezogen auf 1 mol α-Fluoracrylsäure). Aus dem

Reaktionsgemisch wird der erhaltene α-Fluoracrylsäureester durch Destillation, vorzugsweise bei einem Druck von 200 bis 1013 mbar, isoliert. Die Destillation word zweckmäßigerweise in Gegenwert von 100 bis 500 ppm eines handelsüblichen Polymerisationsinhibitors, z.B. Hydrochinon oder Hydrochinonmonomethylether, durchgeführt. Die Reinigung erfolgt durch erneute Destillation oder Umkristallisation.

Für die Veresterung der α-Fluoracrylsäureester wird ein Alkohol der Formel (3) verwendet

(3) $$HO—C(CF_3)_2—R,$$

in der R ein Wasserstoffatom, ein Deuteriumatom, ein Halogenatom (vorzugsweise ein Fluoratom), einen aliphatischen Rest (vorzugsweise einen Alkylrest, der gegebenenfalls Deuteriumatome oder Fluoratome aufweist) mit 1 bis 4 Kohlenstoffatomen oder einen aromatischen Rest (vorzugsweise einen Phenylrest, der gegebenenfalls mit einem oder mehreren Halogenatomen oder einem oder mehreren niederen Alkyloder Alkoxyresten substituiert ist) mit 4 bis 10 Kohlenstoffatomen bedeutet. Geeignete Alkohole sind beispielsweise 1,1,1,3,3,3-Hexafluor-2-propanol, 1,1,1,3,3,3-Hexafluor-2-propanol-$D_2$, Perfluor-2-propanol, Perfluor-tert.-butanol, Perfluor-1,1-dimethyl-propanol, 1,1,1,3,3,3-Hexafluor-2-methyl-2-propanol, 1,1-Bis)trifluormethyl)-propanol, 1,1-Bis(trifluormethyl)propanol-$D_5$, 1,1,1,3,3,3-Hexafluor-2-methyl-2-propanol, Perfluor-2,3-dimethyl-2-butanol, Hexafluor-2-phenyl-2-propanol, Hexafluor-2-(4)-fluorphenyl)-2-propanol, Hexafluor-2-(3,4-dimethyl-phenyl)-2-propanol, Hexafluor-2-(4-methoxyphenyl)-2-propanol, Hexafluor-2-(2-furyl)-2-propanol, Hexafluor-2-(2-thienyl)-2-propanol. Der Alkohol wird gegebenenfalls in Form eines Alkalialkoholats, vorzugsweise eines Natriumalkoholats oder Kaliumalkoholats, eingesetzt.

Die erfindungsgemäßen Ester der α-Fluoracrylsäure sind bei Raumtemperatur farblose Flüssigfkeiten oder Feststoffe. Sie sind polymerisierbar und eignen sich als Ausgangsmaterial zur Herstellung von Fluorpolymeren. Sie sind auch mit anderen Vinylverbindungen copolymerisierbar. Solche besonders geeigneten Vinylverbindungen sind Ester der Methacrylsäure und Ester der α-Fluoracrylsäure, vorzugsweise Methacrylsäurealkylester und α-Fluoracrylsäurealkylester mit jeweils 1 bis 6 Kohlenstoffatomen im Alkylrest. Beispiele hierfür sind insbesondere Methacrylsäuremethylester und α-Fluoracrylsäuremethylester. Das Gewichtsverhältnis von α-Fluoracrylsäureester zu den als Comonomeren eingesetzten anderen Vinylverbindungen beträgt im allgemeinen 60:40 bis 99:1, vorzugsweise 65:35 bis 75:25.

Die Polymerisations wird in üblicher Weise, vorzugsweise in Masse, mit Hilfe eines radikalisch wirksamen Initiators durchgeführt. Geeignete Initiatoren sind beispielsweise Azoverbindungen wie Azobisisobutyronitril und organische Peroxide wie tert.-Butylperoxid, tert.-Butylperoctoat, tert.-Butylperoxyisopropylcarbonat, tert.-Butylhydroperoxid und tert.-Butylperoxyisobutyrat. Die Menge des Initiators liegt im Bereich von 0,01 bis 3, vorzugsweise 0,03 bis 0,3 Mol pro 100 Mol des oder der Monomeren. Vorteilhaft ist die Durchführung der Polymerisation in Gegenwart eines Kettenübertragungsmittels (Reglers). Hierfür eignen sich insebesondere Merkaptane wie Butylmerkaptan, tert.-Butylmerkaptan, Propylmerkaptan, Phenylmerkaptan und tert.- Hexylmerkaptan sowie Ester der Merkaptoessigsäure, z.B. Ethylmerkaptoacetat und Ethylenglykolbis(merkaptoacetat). Die Polymerisationstemperatur beträgt 60 bis 150°C, vorzugsweise 80 bis 130°C.

Es ist empfehlenswert, das Reaktionsgemisch vor Beginn der Polymerisation zu entgasen. Hierzu wird das Reaktionsgemisch aus Monomeren, Initiator und gegebenenfalls Regler in einem Reaktor zunächst auf eine Temperatur von mindestens −100°C abgekühlt, dann wird der Reaktor evakuiert un din verschlossenem Zustand auf eine Temperatur von 0 bis 25°C erwärmt; dieser Vorgant kann mehrere Male wiederholt werden.

Das erfindungsgemäße Polymer entsteht in Form einer glasklaren Masse, die thermoplastisch verformbar ist. Es ist daher vor allem als Material zur Herstellung von transparenten Gegenständen geeignet, z.B. Resistmaterialen, Linsen und Lichtwellenleiter. Die spektrale Durchlässigkeit des Polymers ist besonders hoch in Wellenlängenbereich von 600 bis 1300 nm. Das Polymer zeigt die nachstehenden charakteristischen Eigenschaften:

Mittler Molmasse 8 000 bis 5 000 000, vorzugsweise 10 000 bis 20 000 (gemessen nach der Lichtstreuungsmethode); Glasumwandlungstemperatur: 95 bis 150°C, vorzugsweise 100 bis 145°C; Zersetzungstemperatur: mindestens 230°C, vorzugsweise 250 bis 300°C.

Die folgenden Beispiele dienen zur näheren Erläuterung der Erfindung. Prozentangaben beziehen sich jeweils auf das Gewicht.

## Beispiel 1

a) In einem 4-Liter-Glaskolben wurden 48 g (0,48 mol) Kaliumhydrogencarbonat in 535 g (6,59 mol) wäßriger Formaldehydlösung (37gewichtsprozentig) gelöst. Zu dieser Lösung wurden 841 g (5,6 mol) α-Fluormalonsäure-dimethylester unter Rühren innerhalb von 3 1/2 Stunden zugetropft; dabei wurde die Temperatur im Bereich von 20 bis 25°C gehalten. Während einer Nachrührzeit von 2 Stunden bei derselben Temperatur fiel α-Hydroxymethyl-α-fluormalonsäuredimethylester als farbloser Feststoff aus. Das Reaktionsgemisch wurde anschlißend mit 2500 g einer wäßrigen gesättigten Ammoniumsulfatlösung versetzt und dann mit Dichlormethan extrahiert. Die Extraktionslösung wurde mit wasserfreiem Natriumsulfat getrocknet. Nach Abdestillieren des Dichlormethans (Badtemperatur 40°C, 25 mbar) wurden 906 g (90 Prozent der Theorie) α-Hydroxymethyl-α-fluoracrylsäure-dimethylester erhalten.

Die Elementaranalyse ergab folgende Werte (Prozent):

| | C | H | F | O |
|---|---|---|---|---|
| berechnet: | 40,0 | 5,04 | 10,54 | 44,4 |
| gefunden: | 39,9 | 5,1 | 10,4 | 44,6 |

b) In einem 2-Liter-Glaskolben, der mit Thermometer und Rührer und über eine Vigreux-Kolonne mit einem Destillationsaufsatz versehen war, wurden 175 g (0,97 mol) α-Hydroxymethyl-α-fluormalonsäure-dimethylester, 750 ml Wasser und 750 ml Salzsäure (36gewichtsprozentig) 2 1/2 Stunden lang zum Sieden erhitzt. Dabei betrug die Temperatur des Reaktionsgemisches 103°C. Anschießend wurde das Reaktionsgemisch destilliert. Das Destillat wurde mit 1 g Hydrochinonmonomethylether versetzt und mit Diethylether extrahiert, und die Extraktionslösung wurde mit wasserfreiem Natriumsulfat getrocknet. In die Lösung wurden dann bei Raumtemperatur 17 g (1 mol) gasförmiges Ammoniak eingeleitet. Der dadurch erhaltene farblose Niederschlag wurde abfiltriert, mit Diethylether gewaschen und bei Raumtemperatur und unter vermindertem Druck getrocknet. Es wurden 70,8 g (68 Prozent der Theorie) Ammonium-α-fluoracrylat erhalten.

Die Elementaranalyse ergab folgende Werte (Prozent):

| | C | H | F | N | O |
|---|---|---|---|---|---|
| berechnet: | 33,6 | 5,6 | 17,7 | 13,1 | 29,9 |
| gefunden: | 33,3 | 5,6 | 17,8 | 13,1 | 29,9 |

c) In einem 2-Liter-Glaskolben, der mit Thermometer, Rührer, Rückflußkühler und Tropftrichter versehen war, wurden 1,2 l o-Xylol, 29 g (0,4 mol) Dimethylformamid und 200 g (1,87 mol) Ammonium-α-fluoracrylat vorgelegt. In diese Vorlage wurden innerhalb von 1 stunde 238 g (2,0 mol) Thionylchlorid zugetropft. Das Reaktionsgemisch wurde dann 2 Stunden lang auf einer Temperatur von 80°C gehalten. Nach anschließender Abkühlung auf eine Temperatur von 0°C wurde dem Gemisch ein Gemisch aus 202 g (2 mol) Triethylamin und 336 g (2 mol) Hexafluorisopropanol innerhalb von 30 Minuten zugefügt. Das resultierende Gemisch wurde noch 30 Minuten nachgerührt, und der entstandene Niederschlag wurde abfiltriert. Das Filtrat wurde mit 1 g Hydrochinonmonomethylether versetzt und rasch bei einem Druck von 266 mbar destilliert. Die im Temperaturbereich von 45 bis 85°C erhaltene Fraktion wurde mit Salzsäure (2,4 molar) und mit Wasser gewaschen, getrocknet und erneut destilliert, und es wurden 232 g (52 Prozent der Theorie) reiner α-Fluoracrylsäure-hexafluorisopropylester erhalten. Dieser stellte eine farblose Flüssigkeit dat mit einem Brechungsindex $n_D^{23}$ = 1,3145, einen Siedepunkt von 46,8°C (bei 213 mbar) und einer Dichte von 1,453 g/cm³ (bei 25°C). Im Wellenlängenbereich von 380 bis 680 nm zeigte er eine mittlere Transmission von mehr als 99 Prozent.

Die Identifizierung erfolgt mit Hilfe des $^1$H—MNR-Spektrums (Tetramethylsilan als Standard) und des $^{19}$F—NMR-Spektrums (Trifluormethan als Standard); Deuterochloroform diente dabei als Lösungsmittel. Für die Verbindung der Formel

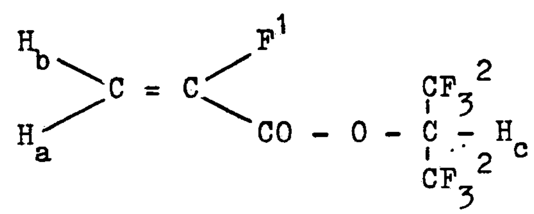

wurden die folgenden chemischen Verschiebungen (δ) und Kopplungskonstanten (J) gemassen:

| | |
|---|---|
| $\delta H_a$ = 5,89 | $J_{H_aH_b}$ = 3,75 Hertz |
| $\delta H_b$ = 5,60 | $J_{H_aF}$ = 41,4 Hertz |
| $\delta H_c$ = 5,81 | $J_{H_bF}$ = 12,2 Hertz |
| $\delta F^1$ = 118,02 | $J_{H_cF}$ = 6,1 Hertz |
| $\delta F^2$ = 73,78 | |

Die Elementarzusammensetzung der Verbindung wurde mit Hilfe der hochauflösenden Massenspektrometrie bestimmt:
berechnet: $M^+$ = 240,0021; gefunden: $M^+$ = 240,0029 m/e

Beispiel 2

a) 150 g (1 mol) α-Fluormalonsäuredimethylester wurden bei einer Temperatur von 25°C tropfenweise

innerhalb einer Stunde zu einem Gemisch aus 96 g einer 35-gewichtsprozentigen wäßrigen Lösung von Formaldehyd (1,1 mol) und 10 g (0,1 mol) Kaliumhydrogencarbonat gegeben. Die Reaktionslösung wurde dann mit dem vierfachen Volumen einer gesättigten wäßrigen Ammoniumsulfatlösung vermischt, und das Gemisch wurde dreimal mit jeweils 150 ml Dichlormethan extrahiert. Die vereinigten Extraktionslösungen wurden über Natriumsulfat getrocknet. Nach Verdampfen des Dichlormethan wurde α-Hydroxymethyl-α-fluormalonsäure-dimethylester als farbloser Feststoff erhalten.

b) 180 g (1 mol) α-Hydroxymethyl-α-fluormalonsäuredimethylester wurden in 1,5 Liter 6 N-Salzsäure 2,5 Stunden lang zum Sieden erhitzt und dadurch dehydratisiert und decarboxyliert. Nach beendeter Gasentwicklung wurde das Reaktionsgemisch bei einem Druck von 700 mbar destilliert, und das Destillat wurde dreimal mit jeweils 150 ml Diethylether extrahiert. Die vereinigten Extraktionslösungen wurden über Natriumsulfat getrocknet. Nach Einleiten von 19 g (1,1 mol) gasförmigen Ammoniak in die Etherlösung wurde das Ammoniumsalz der α-Fluoracrylsäure als farbloser Feststoff erhalten.

c) 107 g (1 mol) des Ammoniumsalzes der α-Fluoracrylsäure wurden mit 14,5 g (0,2 mol) Dimethylformamid und 0,6 l Xylol vermischt, und das Gemisch wurde innerhalb einer Stunde tropfenweise mit 131 g (1,1 mol) Thionylchlorid versetzt; anschließend wurde das Gemisch zwei Stunden lang auf eine Temperatur von 80° erwärmt. Nach Abkühlen auf eine Temperatur von 0°C wurde dem Reaktionsgemisch ein Gemisch aus 204 g (1,1 mol) Tributylamin und 185 g (1,1 mol) Hexafluorisopropanol innerhalb von 30 min zugefügt.

Dann wurde das Gemisch noch eine Stunde lang bei einer Temperatur von 30°C gerührt und schließlich filtriert. Das Fltrat wurde bei einem Druck von 270 mbar destilliert, un es wurde α-Fluoracrylsäurehexafluorisopropylester als farblose Flüssigkeit erhalten.

## Beispiel 3

Beispiel 2 wurde wiederholt, wobei in Anschnitt c) an Stelle von 185 g Hexafluorisopropanol nun 187,1 g (1,1 mol) Dideuterohexafluoroisopropanil verwendet werden.

Nach Destillation bei einem Druck von 213 mbar wurden 132,5 g (50 Prozent der Theorie) α-Fluoracrylsäuredeuterohexafluorisopropylester als farblose Flüssigkeit mit einem Siedepunkt von 47°C erhalten.

## Beispiel 4

a) In einem 1-Liter-Glaskolben wurden 100 g (0,934 mol) Ammonium-o-fluoracrylat (erhalten nach Beispiel 1) in einem Gemisch aus 600 g Mesitylen und 15 ml Dimethylformamid dispergiert und innerhalb einer Stunde mit 119 g (1,0 mol) Thionylchlorid versetzt. Das erhaltene Gemisch wurde auf eine Temperatur von 80°C erhitzt und 2 Stunden lang unter Rühren bei dieser Temperatur gehalten. Die nach Erkalten auf Raumtemperatur erhaltene Flüssigkeit wurde bei vermindertem Druck destilliert, und die bis 100°C/160 mbar erhaltene Fraktion wurde nochmals bei Normaldruck destilliert. Es wurden 67 g (66 Prozent der Theorie) α-Fluoracrylsäurechlorid mit einem Siedepunkt von 65 bis 67°C erhalten.

b) In eine Suspension von 5,34 g (0,092 mol) getrocknetem Kaliumfluorid in 25 ml trockenem Diethylenglykoldimethylether wurden bei einer Temperatur von 25°C 15,3 g (0,092 mol) Hexafluoraceton innerhalb von 30 Minuten unter Rühren eingeleitet, wobei sich das Kaliumfluorid auflöste. Nach einer Nachrührzeit von 2 Stunden wurde nicht umgesetztes Hexafluoraceton unter vermindertem Druck (Wasserstrahlpumpe) abdestilliert. In die verbleibende Lösung wurden bei einer Temperatur von 25°C unter Rühren innerhalb von 5 Minuten 10 g (0,092 mol) α-Fluoracrylsäurechlorid zugetropft, wobei ein farbloser Feststoff ausfiel. Das Reaktionsgemisch wurde noch 80 Minuten lang bei einer Temperatur von 25°C gerührt, dann mit 0,005 g Hydrochinonmonomethylether versetzt und anschließend bei einem Druck von 40 mbar destilliert. Es wurden 17,6 g (74 Prozent der Theorie) α-Fluoracrylsäureperfluorisopropylester mit einem Siedepunkt von 40°C (bei 165 mbar) erhalten.

## Beispiel 5

Zu einer Lösung von 62 g (0,571 mol) α-Fluoracrylsäurechlorid (erhalten nach Beispiel 4) in 100 ml trockenem Diethylether wurde bei einer Temperatur von 25°C eine Lösung von 213,6 g (0,571 mol) Kaliumperfluor-2,3-dimethyl-2-butanolat in 250 ml trockenem Diethylether innerhalb von 1 Stunde unter Rühren zugetropft, wobei ein farbloser Feststoff ausfiel. Das Reaktionsgemisch wurde noch 1 Stunde lang bei einer Temperatur von 25°C gerührt, und der Festoff wurde abfiltriert. Das Filtrat wurde nach Zugabe von 0,01 g Hydrochinonmonomethylether unter vermindertem Druck destilliert. Es wurden 167 g (72 Prozent der Theorie) α-Fluoracrylsäureperfluor-2,3-dimethyl-2-butylester mit einem Siedepunkt von 43°C (bei 20 mbar) erhalten.

## Beispiel 6

Zu einer Lösung von 22,5 g (0,207 mol) α-Fluoracrylsäurechlorid (erhalten nach Beispiel 4) in 100 ml trockenem Dichlormethan wurde bei einer Temperatur von 25°C ein Gemisch aus 50 g (0,205 mol) 2-Phenyl-hexafluorisopropanol und 20,8 g (0,205 mol) Triethylamin innerhalb von 20 Minuten unter Rühren zugetropft. Das Reaktionsgemisch wurde anschließend noch 90 Minuten lang auf einer Temperatur von 45°C gehalten. Nach Abkühlen des Gemisches auf 25°C wurde der gebildete Feststoff abfiltriert. Das Filtrat wurde nach Zugabe von 0,005 g Hydrochinonmonomethylether unter vermindertem Druck destilliert. Es wurden 38,3 g (58 Prozent der Theorie) α-Fluoracrylsäure-2-phenyl-hexafluorisopropylester mit einem Siedepunkt von 66°C (bei 5 mbar) erhalten.

Beispiel 7

Zu einer Lösung von 18 g (0,166 mol) α-Fluoracrylsäurechlorid (erhalten nach Beispiel 4) in 25 ml trockenem Diethylether wurde bei einer Temperatur von 25°C eine Lösung von 40 g (0,153 mol) 2-(4-Fluorphenyl)-hexafluor-2-propanol und 15,5 g (0,153 mol) Triethylamin in 75 ml trockenem Diethylether innerhalb von 45 Minuten unter Rühren zugetropft. Das Reaktionsgemisch wurde anschließend noch 2 Stunden lang bei einer Temperatur von 25°C gerührt. Der entstandene Feststoff wurde abfiltriert. Das Filtrat wurde Zugabe von 0,01 g Hydrochinonmonomethylether unter vermindertem Druck destilliert. Es wurden 42,35 g (83 Prozent der Theorie) α-Fluoracrylsäure-2-(4-fluorophenyl)-hexafluor-2-propylester mit einem Siedepunkt von 70 bis 73°C (bei 0,5 mbar) erhalten.

Beispiel 8

Zu einer Lösung von 8.7 g (0,08 mol) α-Fluoracrylsäurechlorid (erhalten nach Beispiel 4) in 25 ml trockenem Diethylether wurde bei einer Temperatur von 20°C eine Lösung von 21,5 g (0,067 mol) 2-(4-Bromphenyl)-hexafluor-2-propanol und 6,8 g (0,067 mol) Triethylamin in 25 ml trockenem Diethylether innerhalb von 30 Minuten unter Rühren zugetropft. Das Reaktionsgemisch wurde anschließend noch 3,5 Stunden lang bei einer Temperatur von 20°C gerührt. Der entstandene Feststoff wurde abfiltriert. Das Filtrat wurde nach Zugabe von 0,005 g Hydrochinonmonomethylether unter vermindertem Druck destilliert. Es wurden 18,8 g (71 Prozent der Theorie) α-Fluoracrylsäure-2-(4-bromphenyl)hexafluor-2-propylester mit einem Siedepunkt von 66 bis 69°C (bei 0,13 mbar) erhalten.

Beispiel 9

Zu einer Lösung von 9.8 g (0,09 mol) α-Fluoracrylsäurechlorid (erhalten nach Beispiel 4) in 25 ml trockenem Diethylether wurde bei einer Temperatur von 20°C eine Lösung von 21 g (0,081 mol) 2-Tolyl-hexafluor-2-propanol und 8,2 g (0,08 mol) Triethylamin in 25 ml trockenem Diethylether innerhalb von 40 Minuten unter Rühren zugetropft. Das Reaktionsgemisch wurde anschließend noch 4 Stunden lang bei einer Temperatur von 20°C gerührt. Der entstandene Feststoff wurde abfiltriert. Das Filtrat wurde nach Zugabe von 0,02 g Hydrochinonmonomethylether unter vermindertem Druck destilliert. Es wurden 19,6 g (73 Prozent der Theorie) α-Fluoracrylsäure-2-tolyl-hexafluor-2-propylester mit einem Siedepunkt von 58 bis 60°C (bei 0,27 mbar) erhalten.

Beispiel 10

Zu einer Lösung von 20 g (0,184 mol) α-Fluoracrylsäurechlorid (erhalten nach Beispiel 4) in 50 ml trockenem Diethylether wurde bei einer Temperatur von 20°C eine Lösung von 27,3 g (0,1 mol) 2-(3,4-dimethylphenyl)-hexafluor-2-propanol und 10,1 g (0,1 mol) Triethylamin in 50 ml trockenem Diethylether innerhalb von 35 Minuten unter Rühren zugetropft. Das Reaktionsgemisch wurde anschließend noch 3,5 Stunden lang bei einer Temperatur von 20°C gerührt. Der entstandene Feststoff wurde abfiltriert. Das Filtrat wurde nach Zugabe von 0,01 g Hydrochinonmonomethylether unter vermindertem Druck destilliert. Es wurden 17,7 g (51 Prozent der Theorie) α-Fluoracrylsäure-2-(3,4-dimethylphenyl)-hexafluor-2-propylester mit einem Siedepunkt von 72 bis 73°C (bei 0,4 mbar) erhalten.

Beispiel 11

Zu einer Lösung von 9,8 g (0,09 mol) α-Fluoracrylsäurechlorid (erhalten nach Beispiel 4) in 25 ml trockenem Diethylether wurde bei einer Temperatur von 20°C eine Lösung von 20 g (0,085 mol) Hexafluor-2-(2-furyl-2-propanol und 8,65 (0,085 mol) Triethylamin in 25 ml trockenem Diethylether innerhalb von 30 Minuten unter Rühren zugetropft. Das Reaktionsgemisch wurde anschließend noch 4 Stunden lang bei einer Temperatur von 20°C gerührt. Der entstandene Feststoff wurde abfiltriert. Das Filtrat wurde nach Zugabe von 0,005 g Hydrochinonmonomethylether unter vermindertem Druck destilliert. Es wurden 15,9 g (61 Prozent der Theorie) α-Fluoracrylsäure-hexafluor-2-(2-furyl)-2-propylester mit einem Siedepunkt von 55 bis 57°C (bei 8 mbar) erhalten.

Beispiel 12

Zu einer Lösung von 9,8 g (0,09 mol) α-Fluoracrylsäurechlorid (erhalten nach Beispiel 4) in 25 ml trockenem Diethylether wurde bei einer Temperatur von 20°C eine Lösung von 20 g (0,08 mol) Hexafluor-2-(2-thienyl)-2-propanol und 8,1 g (0,08 mol) Triethylamin in 25 ml trockenem Diethylether innerhalb von 30 Minuten unter Rühren zugetropft. Das Reaktionsgemisch wurde anschließend noch 3 Stunden lang bei einer Temperatur von 20°C gerührt. Der entstandene Feststoff wurde abfiltriert. Das Filtrat wurde nach Zugabe von 0,005 g Hydrochinonmonomethylether unter vermindertem Druck destilliert. Es wurden 13,6 g (53 Prozent der Theorie) α-Fluoracrylsäure-hexafluor-2-(2-thienyl-propylester mit einem Siedepunkt von 71 bis 72°C (bei 0,5 mbar) erhalten.

Beispiel 13

Eine Lösung aus 100 g α-Fluoracrylsäure-hexafluorisopropylester (FAFP), 0,13 g Azobisisobutyronitril (AIBN) und 0,33 g Butylmerkaptan wurde durch ein Membranfilter (Porenweite 45 nm) in ein Glasgefäß filtriert und sorgfältig entgast. Die Entgasung erfolgte dadurch, daß das Reaktionsgemisch zunächst mit Hilfe von flüssigem Stickstoff eingefroren wurde, das Glasgefäß dann evakuiert (0,001 mbar) und auf

Raumtemperatur erwärmt wurde, und dieser Vorgang fünfmal wiederholt wurde. Anschließend wurde das Glasgefäß verschlossen und das entgaste Reaktionsgemisch zunächst 3 Stunden lang auf eine Temperatur von 60°C und dann 4 Stunden lang auf eine Temperatur von 80°C erwärmt. Nach Abkühlen des Reaktionsgemisches auf Raumtemperatur wurde eine glasklare Polymermasse erhalten, an der die nachstehenden Eigenschaften gemessen wurden:

Mittlere Molmasse: 150 000
Glasumwandlungstemperatur: 108,5°C
Zersetzungstemperatur: 250°C
Schmelzindex (230°C; 3,8 kg): 8 g/10 min
Restmonomergehalt: 0,2%
Brechungsindex $n_D^{23}$: 1,355

Beispiel 14

Eine Lösung aus 50 g Methanol, 50 g FAFP, 0,03 g AIBN und 3 g Butylmerkaptan wurde analog Beispiel 13 filtriert und entgast. Das entgaste Reaktionsgemisch wurde dann 20 Stunden lang auf eine Temperatur von 60°C erwärmt. Das Reaktionsgemisch wurde nach Abkühlen auf Raumtemperatur mit 400 ml Aceton versetzt, und das erhaltene Gemisch wurde in 6 l Hexan gegossen. Das gefällte Polymer wurde von der Flüssigkeit angetrennt und 6 Stunden lang bei einer Temperatur von 70°C getrocknet. Es wurden 40 g (80 Prozent der Theorie) eines Polymers erhalten, an dem die nachstehenden Eigenschaften gemassen wurden:

Mittlere Molmasse: 10 000
Glasumwandlungstemperatur: 102°C
Zersetzungstemperatur: 250°C

Beispiele 15 bis 20

Lösungen aus unterschiedlichen Mengen von FAFP und Methylmethacrylat (MMA) mit jeweils 0,1 g AIBN und 0,15 g Butylmerkaptan wurden analog Beispiel 13 filtriert und entgast. Die entgasten Reaktionsgemische wurden jeweils 30 min lang auf eine Temperatur von 60°C erwärmt und nach Abkühlen auf Raumtemperatur mit 300 ml Aceton versetzt. Die jeweils erhaltenen Gemische wurden in 5 l Hexan gegossen, und die gefällten Copolymeren wurden von der Flüssigkeit getrennt und 6 Stunden lang bei einer Temperatur von 70°C getrocknet.

Die jeweilige Zusammensetzung des Monomergemisches und des Copolymers sowie die Glasumwandlungstemperatur (Tg) des Copolymers sind aus Tablelle 1 ersichtlich.

Tabelle 1

| Beispiel | Gewichtsverhältnis MMA : FAFP Monomergemisch (g) | Gewichtsverhältnis MMA : FAFP Copolymer (%) | Tg (°C) |
|---|---|---|---|
| 15 | 85 : 15 | 70 : 30 | 119 |
| 16 | 71 : 29 | 58 : 42 | 110 |
| 17 | 48 : 52 | 43 : 57 | 107 |
| 18 | 38 : 62 | 38 : 62 | 105 |
| 19 | 29 : 71 | 34 : 66 | 103 |
| 20 | 17 : 83 | 28 : 72 | 97 |

Beispiele 21 bis 23

Lösungen aus unterschiedlichen Mengen von FAFP und α-Fluoracrylsäuremethylester (FAM) mit jeweils 0,1 g AIBN und 0,15 g Butylmerkaptan wurden analog Beispiel 13 filtriert und entgast. Die entgasten Reaktionsgemsche wurden jeweils 30 min lang auf eine Temperatur von 60°C erwärmt und nach Abkühlen auf Raumtemperatur mit 300 ml Aceton versetzt. Die jeweils erhaltenen Gemische wurden in 5 l Hexan gegossen, und die gefällten Copolymeren wurden von der Flüssigkeit getrennt und 6 Stunden lang bei einer Temperatur von 70°C getrocknet.

Die jeweilige Zusammensetzung des Monomergemisches und des Copolymers sowie die Glasumwandlungstemperatur des Copolymers sind aus Tablelle 2 ersichtlich.

Tabelle 2

| Beispiel | Gewichtsverhältnis FAM : FAFP Monomergemisch (g) | Copolymer (%) | Tg (°C) |
|---|---|---|---|
| 21 | 32 : 68 | 21 : 79 | 133 |
| 22 | 52 : 48 | 34 : 66 | 142 |
| 23 | 74 : 26 | 55 : 45 | 140 |

**Patentansprüche für die Vertragsstaaten: BE CH DE FR GB IT LI LU NL SE**

1. α-Fluoracrylsäureester der Formel (1)

(1) $CH_2=CF—CO—O—C(CF_3)_2—R$,

in der R ein Wasserstoffatom, ein Deuteriumatom, ein Halogenatom, einen aliphatischen Rest mit 1 bis 4 Kohlenstoffatomen oder einem aromatischen Rest mit 4 bis 10 Kohlenstoffatomen bedeutet.

2. α-Fluoracrylsäureester der Formel (2)

(2) $CH_2=CF—CO—O—C(CF_3)_2—H$.

3. Verfahren zur Herstellung α-Fluoracrylsäureesters, dadurch gekennzeichnet, daß in einem ersten Verfahrensschritt α-Fluormalonsäure-dimethylester mit Formaldehyd umgesetzt wird, dann in einem zweiten Verfahrensschritt der erhaltene hydroxymethylierte α-Fluormalonsäure-dimethylester hydrolysiert, decarboxyliert und dehydratisiert wird und anschließend in einem dritten Verfahrensschritt die erhaltene α-Fluoracrylsäure, gegebenenfalls in Form eines Säurehalogenids, mit einem Alkohol der Formel (3)

(3) $HO—C(CF_3)_2—R$,

in der R die bei Formel (1) angegebene Bedeutung hat, gegebenenfalls in Form eines Alkalialkoholats, verestert wird.

4. Verfahren nach Anspruch 3, dadurch gekennzeichnet, daß der erste Verfahrensschritt bei einer Temperatur von 5 bis 40°C, der zweite Verfahrensschritt bei einer Temperatur von 90 bis 110°C und der dritte Verfahrenschritt bei einer Temperatur von 0 bis 30°C durchgeführt werden.

5. Fluorhaltiges Polymer, dadurch gekennzeichnet, daß es im wesentlichen aus Monomereinheiten besteht, die sich von einem α-Fluoracrylsäureester der Formel (a) ableiten.

6. Polymer nach Anspruch 5, dadurch gekennzeichnet, daß es Monomereinheiten enthält, die sich von verschiedenen α-Fluoracrylsäureestern der Formel (a) ableiten.

7. Polymer nach Anspruch 5, dadurch gekennzeichnet, daß es sowohl Monomereinheiten, die sich von einem α-Fluoracrylsäureester der Formel (1) ableiten, als auch Monomereinheiten, die sich von einer anderen, copolymerisierbaren Vinylverbindung ableiten, enthält.

8. Verfahren zur Herstellung eines fluorhaltigen Polymers durch radikalisch initiierte Polymerisation eines fluorhaltigen Monomers, dadurch gekennzeichnet, daß ein α-Fluoracrylsäureester der Formel (1), gegebenenfalls im Gemisch mit einer anderen, copolymerisierbaren Vinylverbindung, bei einer Temperatur von 60 bis 150°C polymerisiert wird.

9. Verwendung eines fluorhaltigen Polymers gemäß Anspruch 5 als Material zur Herstellung von transparenten Gegenständen.

**Patentansprüche für den Vertragsstaat: AT**

1. Verfahren zur Herstellung eines α-Fluoracrylsäureesters, dadurch gekennzeichnet, daß in einem ersten Verfahrensschritt α-Fluormalonsäure-dimethylester mit Formaldehyd umgesetzt wird, dann in einem zweiten Verfahrenschritt der erhaltene hydroxymethylierte α-Fluormalonsäure-dimethylester hydrolysiert, decarboxyliert und dehydratisiert wird und anschließend in einem dritten Verfahrensschritt die erhaltene α-Fluoracrylsäure, gegebenenfalls in Form eines Säurehalogenids, mit einem Alkohol der Formel (3)

(3) $HO—C(CF_3)_2—R$,

in der R ein Wasserstoffatom, ein Deuteriumatom, ein Halogenatom, einen aliphatischen Rest mit 1 bis 4

Kohlenstoffatomen oder einem aromatischen Rest mit 4 bis 10 Kohlenstoffatomen bedeutet, gegebenenfalls in Form eines Alkalialkoholats, verestert wird.

2. Verfahren nach Anspruch 1, dadurch gekennzeichnet, daß der erste Verfahrensschritt bei einer Temperatur von 5 bis 40°C, der zweite Verfahrensschritt bei einer Temperatur von 90 bis 110°C und der dritte Verfahrensschritt bei einer Temperatur von 0 bis 30°C durchgeführt werden.

3. Verfahren zur Herstellung eines fluorhaltigen Polymers durch radikalisch initiierte Polymerisation eines fluorhaltigen Monomers, dadurch gekennzeichnet, daß ein α-Fluoracrylsäureester der Formel (1)

(1) $$CH_2{=}CF{-}CO{-}O{-}C(CF_3)_2{-}R,$$

in der R ein Wasserstoffatom, ein Deuteriumatom, ein Halogenatom, einen aliphatischen Rest mit 1 bis 4 Kohlenstoffatomen oder einem aromatischen Rest mit 4 bis 10 Kohlenstoffatomen bedeutet, gegebenenfalls im Gemisch mit einer anderen, copolymerisierbaren Vinylverbindung, bei einer Temperatur von 60 bis 150°C polymerisiert wird.

9. Verwendung eines fluorhaltigen Polymers gemäß Anspruch 3, als Material zur Herstellung von transparenten Gegenständen.

**Revendications pour les Etats contractants: BE CH DE FR GB IT LI LU NL SE**

1. Esters de l'acide α-fluoroacrylique de formule (1) ci-dessous:

(1) $$CH_2{=}CF{-}CO{-}O{-}C(CF_3)_2{-}R,$$

dans laquelle R désigne un atome d'hydrogène, de deutérium ou d'halogène ou bien un radical aliphatique en $C_1$—$C_4$ ou un radical aromatique en $C_4$—$C_{10}$.

2. Esters de l'acide α-fluoroacrylique de formule (2):

(2) $$CH_2{=}CF{-}CO{-}O{-}C(CF_3)_2{-}H.$$

3. Procédé de préparation d'esters de l'acide α-fluoroacrylique, procédé caractérisé en ce que dans une première étape on fait réagir l'α-fluoromalonate de diméthyle avec le formaldéhyde, dans une seconde étape on hydrolyse, décarboxyle et déshydrate l'α-fluoromalonate de diméthyle hydroxyméthylé ainsi formé, et dans une troisième étape on estérfie l'acide α-fluoroacrylique obtenu, éventuellement sous la forme d'un halogénure d'acide, avec un alcool de formule (3)

(3) $$HO{-}C(CF_3)_2{-}R,$$

R ayant la signification donnée pour la formule (1) de la revendication 1, le cas échéant sous la forme d'une alcoolate de métal alcalin.

4. Procédé selon la revendication 3, caractérise en ce que la première étape se fait à une température de 5 à 40°C, la seconde étape à une température de 90 à 110°C et la troisième étape à une température de 0 à 30°C.

5. Polymères fluorés, caractérisés en ce qu'ils sont essentiellement formés de motifs de monomères provenant d'un ester de l'acide α-fluoroacrylique de formule (1), selon la revendication 1.

6. Polymères selon la revendication 5, caractérisés en ce qu'ils comportent des motifs monomères de divers esters α-fluoroacryliques de formule (1).

7. Polymères selon la revendication 5, caractérisés en ce qu'ils comportent à la fois des motifs monomères d'un ester α-fluoroacrylique de formule (1) et des motifs monomères d'un autre composé vinylique copolymérisable avec cet ester.

8. Procédé de préparation de polymères fluorés par polymérisation d'un monomère fluoré induite par des radicaux libres, procédé caractérise en ce que l'on polymérise un ester α-fluoroacrylique de formule (1) selon la revendication 1, éventuellement avec un autre composé vinylique copolymérisable avec cet ester, à une température de 60 à 150°C.

9. L'emploi des polymères fluorés de la revendication 5 comme matériaux de fabrication d'articles transparents.

**Revendications pour l'Etat contractant: AT**

1. Procédé de préparation d'esters de l'acide α-fluoroacrylique, procédé caractérisé en ce que dans une première étape on fait réagir l'α-fluoromalonate de diméthyle avec le formaldéhyde, dans une seconde étape on hydrolyse, décarboxyle et déshydrate l'α-fluoromalonate de diméthyle hydroxyméthylé formé, et dans une troisième étape on estérfie l'acide α-fluoroacrylique obtenu, éventuellement sous la forme d'un halogénure d'acide, avec un alcool de formule (3)

(3) $$HO{-}C(CF_3)_2{-}R,$$

R désigne un atome d'hydrogène, de deutérium ou, d'halogène ou bien un radical aliphatique en $C_1—C_4$ ou un radical aromatique en $C_4—C_{10}$, l'alcoolate pouvant être éventuellement sous la forme d'un alcoolate de métal alcalin.

2. Procédé selon la revendication 1, caractérisé en ce que la première étape se fait à une température de 5 à 40°C, la seconde étape à une température de 90 à 110°C et la troisième étape à une température de 0 à 30°C.

3. Procédé de préparation de polymères fluorés par polymérisation d'un monomère fluoré induite par des radicaux libres, procédé caractérisé en ce que l'on polymérise un ester de l'acide α-fluoroacrylique de formule (1)

(1) $$CH_2=CF—CO—O—C(CF_3)_2—R,$$

(dans laquelle R désigne un atome d'hydrogène, de deutérium ou d'halogène ou bien un radical aliphatique en $C_1—C_4$ ou un radical aromatique en $C_4—C_{10}$), le cas échéant avec un autre composé vinylique copolymérisable evec cet ester, à une température de 60 à 150°C.

4. L'emploi des polymères fluorés obtenus selon la revendication 3 comme matériaux de fabrication d'articles transparents.

**Claims for the Contracting States: BE CH DE FR GB IT LI LU NL SE**

1. An α-fluoroacrylic ester of the formula (1)

(1) $$CH_2=CF—CO—O—C(CF_3)_2—R,$$

in which R denotes a hydrogen atom, a deuterium atom, a halogen atom, an aliphatic radical having 1 to 4 carbon atoms for an aromatic radical having 4 to 10 carbon atoms.

2. The α-fluoroacrylic ester of the formula (2)

(2) $$CH_2=CF—CO—O—C(CF_3)_2—H.$$

3. A process for preparing an α-fluoroacrylic ester, which comprises reacting in a first process step a dimethyl α-fluoromalonate with formaldehyde, then in a second process step subjecting the resulting hydroxymethylated dimethyl α-fluoromalonate to hydrolysis, decarboxylation and dehydration and subsequently, in a third process step, esterifying the resulting α-fluoroacrylic acid, if desired in the form of an acid halide, with an alcohol of the formula (3)

(3) $$HO—C(CF_3)_2—R,$$

in which R has the meaning indicated in the case of formula (1), if desired in the form of an alkali metal alcoholate.

4. The process as claimed in claim 3, wherein the first process step is carried out at a temperature of 5 to 40°C, the second process step at a temperature of 90 to 110°C and the third process step at a temperature of 0 to 30°C.

5. A flourine-containing polymer consisting essentially of monomer units which are derived from an α-fluoroacrylic ester of the formula (1).

6. The polymer as claimed in claim 5, which contains monomer units which are derived from different α-fluoroacrylic esters of the formula (1).

7. The polymer as claimed in claim 5, which contains not only monomer units which are derived from an α-fluoroacrylic ester of the formula (1) but also monomer units which are derived from another copolymerizable vinyl compound.

8. A process for preparing a fluorine-containing polymer by a free-radical initiated polymerization of a fluorine-containing monomer, which comprises polymerizing an α-fluoroacrylic ester of the formula (1), if desired in mixture with another copolymerizable vinyl compound, at a temperature of 60 to 150°C.

9. Use of a fluorine-containing polymer as claimed in claim 5, as a material for manufacturing transparent articles.

**Claims for the Contracting State: AT**

1. A process for preparing an α-fluoroacrylic ester, which comprises reacting in a first process step a dimethyl α-fluoromalonate with formaldehyde, then in a second process step subjecting the resulting hydroxymethylated dimethyl α-fluoromalonate to hydrolysis, decarboxylation and dehydration and subsequently, in a third process step, esterifying the resulting α-fluoroacrylic acid, if desired in the form of an acid halide, with an alcohol of the formula (3)

(3) $$HO—C(CF_3)_2—R,$$

in which R denotes a hydrogen atom, a deuterium atom, a halogen atom, an aliphatic radical having 1 to 4 carbon atoms for an aromatic radical having 4 to 10 carbon atoms, if desired in the form of an alkali metal alcoholate.

2. The process as claimed in claim 1, wherein the first process step is carried out at a temperature of 5 to 40°C, the second process step at a temperature of 90 to 110°C and the third process step at a temperature of 0 to 30°C.

3. A process for preparing a fluorine-containing polymer by a free-radical initiated polymerization of a fluorine-containing monomer, which comprises polymerizing an α-fluoroacrylic ester of the formula

(1) $$CH_2=CF—CO—O—C(CF_3)_2—R$$

in which R denotes a hydrogen atom, a deuterium atom, a halogen atom, an aliphatic radical having 1 to 4 carbon atoms or an aromatic radical having 4 to 10 carbon atoms, if desired in mixture with another copolymerizable vinyl compound, at a temperature of 60 to 150°C.

4. Use of a fluorine-containing polymer prepared as claimed in claim 3, as a material for manufacturing transparent articles.